# EUROPEAN PATENT SPECIFICATION

(11) **EP 3 919 901 B1**
(45) Date of publication and mention of the grant of the patent: **27.12.2023**
(21) Application number: 20178348.7
(22) Date of filing: 04.06.2020
(51) Int. Cl.: F25D 29/00, G01N 33/12, G01N 33/02, A23B 7/148, F25D 17/04

(54) **SENSING DEVICE FOR A FOOD STORAGE CONTAINER**
ERFASSUNGSVORRICHTUNG FÜR EINEN LEBENSMITTELVORRATSBEHÄLTER
DISPOSITIF DE DÉTECTION DESTINÉ À UN RÉCIPIENT DE STOCKAGE D'ALIMENTS

(43) Date of publication of application: 08.12.2021
(73) Proprietor: BSH Hausgeräte GmbH, 81739 München (DE)
(72) Inventor: Sagoo, Kiran Pal, 82256 Fürstenfeldbruck (DE); Hottner, Lena, 87439 Kempten (DE); Sell, Melina, 33689 Bielefeld (DE)

(56) References cited:
- WO-A1-2017/097683
- KR-A- 20080 104 705
- US-A1- 2008 225 154
- US-A1- 2015 285 774
- US-A1- 2016 341 709
- US-A1- 2017 219 279
- US-A1- 2019 033 282
- US-A1- 2019 387 375
- US-B1- 6 337 469

## Description

The present document relates to a sensing device for a food storage container, which is configured to provide information regarding the spoilage or freshness status of food stored within the storage container.

Food is often stored within a refrigerator using a storage container, notably a storage box, which comprises a receptacle that is covered with a lid. Even though the storage container may be made up of a transparent material such that a user may verify the status of stored food visually, it often happens that food spoils when stored within a storage container.

The documents US 2019/033282 A1, US 2016/341709 A1, WO 2017/097683 A1, US 2019/387375 A1, US 6337 469 B1, US 2008/225154 A1, US 2015/285774 A1 and KR 2008 0104705 A disclose relating sensing devices and storage containers.

The present document addresses the technical problem of providing efficient, comfortable and reliable means for preventing food from spoiling, when being stored within a storage container. The technical problem is solved by the independent claim. Preferred examples are described in the dependent claims.

According to an aspect, a sensing device for determining the freshness or spoilage status of a food item (e.g. a fruit, a vegetable, a piece of meat, a piece of fish, etc.) at least partially placeable or at least partially placed within a receptacle (e.g. a box, a glass, a pot, etc.) of a storage container is described. The freshness status may comprise a first status indicating that the food item is not-yet spoiled, a second status indicating that the food item will soon be spoiled (e.g. within the next 24 or 48 hours) and/or a third status indicating that the food item is spoiled.

The storage container may exhibit a storage volume between 0,5 liters and 5 liters. The storage container may comprise the receptacle for receiving the food item. Furthermore, the storage container may comprise a lid for covering and/or for closing the receptacle. For this purpose, the lid may be placed on the rim of the receptacle.

The sensing device is designed in a self-sustained manner, i.e. the sensing device is designed as an independent device, which is configured to be operated in a stand-alone manner. In particular, the sensing device may be built as a single connected and/or discontiguous entity (e.g. within a single or multiple physically connected housings).

The sensing device comprises a gas sensor, notably a volatile organic compounds (VOC) sensor, which is configured to capture sensor data regarding gas comprised within the receptacle, when the gas sensor is placed within the receptacle. The gas sensor may be configured to provide sensor data in a periodic manner (e.g. every 30 minutes). The sensor data may be indicative of the gas (notably of the chemical composition of the gas) comprised within the receptacle. The gas sensor may be provided with electrical power from a battery unit (e.g. a lithium battery) of the sensing device.

Furthermore, the sensing device comprises a user interface, which is configured to allow a user to indicate the type of the food item that is placed or is intended to be placed within the receptacle. In particular, the user interface may allow a user to select a food item type from a list of pre-determined food item types. Example food item types are: one or more different types of fruits (e.g. bananas, apples, etc.); one or more different types of vegetables (lettuce, eggplant, etc.); one or more different types of meat (e.g. chicken, beef, pork, etc.) and/or one or more types of fish (filet, entire fish, etc.). The indication of the food item type may be or may comprise an identifier allowing the food item type to be identified from the list of pre-determined food item types.

In addition, the sensing device comprises a control unit, which is configured to enable determination of the freshness or spoilage status of the food item based on the sensor data provided by the gas sensor and based on the indication of the type of the food item provided by the user interface. In particular, the control unit may be configured to directly determine the freshness status based on the sensor data and based on the indication of the food item type (e.g. using supporting data provided by an external server). Alternatively, or in addition, the control unit may be configured to provide the sensor data and the indication of the food item type to an external computing device (e.g. a server) for enabling the external computing device to determine the freshness status of the food item, which is placed within the receptacle.

By providing a user interface for selecting the appropriate food item type directly at the sensing device, the freshness status of a food item may be determined in an efficient, comfortable and precise manner.

The sensing device may comprise a communication unit (e.g. a Wireless LAN transmitter/receiver) configured to provide a communication interface, notably a wireless communication interface (e.g. WLAN), to a remote computing entity (e.g. a cloud server or a smart device such as a smartphone of a user). The control unit may be configured to transmit the sensor data and the indication of the type of the food item using the communication unit, in order to enable the remote computing entity to determine the freshness status of the food item. By providing the sensor data and the indication of the food item type to an external computing entity, the freshness status of the food item may be determined in a particularly precise manner.

The user interface comprises a rotatable wheel which is configured to be turned by the user to a plurality of different type segments (e.g. different segments of a cake) for selecting one of a corresponding plurality of different food item types. By way of example, the wheel may comprise between 5 and 15 different type segments. The plurality of different type segments may comprise a plurality of different symbols for indicating the corresponding plurality of different food item types.

The control unit is configured to determine the indication of the type of the food item based on the type segment that the rotatable wheel has been turned to. In particular, the sensing device may comprise a button, which is configured to be actuated, notably touched and/or pushed, by the user. The control unit may be configured to detect an actuation of the button. Furthermore, the control unit may be configured, in reaction to detecting an actuation of the button, to determine the indication of the type of the food item based on the type segment that the rotatable wheel has been turned to.

By providing a wheel or a twist dial for selecting the appropriate food item type, the indication of the food item type may be selected by the user in a particularly comfortable and reliable manner.

The sensing device comprises a first module comprising the gas sensor and a second module comprising the control unit and the user interface. Each module may be integrated within a separate housing or may comprise a separate housing. The first module and the second module, notably the housing of the first module and the housing of the second module, are coupled with one another via a flexible strap. The flexible strap is configured to be placed over a rim of the receptacle such that the first module is placed inside the receptacle and the second module is placed outside the receptacle. For this purpose, the strap may pliable but preferably not extensible.

Providing a sensing device which comprises two separate modules which are interconnected using a flexible strap, allows the sensing device to be placed onto the receptacle of a storage container in a flexible and reliable manner.

The sensing device may comprise a plurality of (electrical) wires within the strap for transmitting the sensor data from the gas sensor to the control unit and/or for providing electrical power from the second module to the gas sensor, thereby enabling a reliable operation of the sensing device.

The first module and the second module may be configured to attract one another through the wall of the receptacle using a magnetic force, such that the first module and the second module are attached to one another. As a result of this, a particularly reliable fixation of the sensing device on a storage container may be provided.

The sensing device (notably the control unit) may be configured to detect whether or not the first module and the second module are attached to one another. This may be detected e.g. using a proximity sensor. Furthermore, the sensing device (notably the control unit) may be configured to automatically start operation of the gas sensor for capturing sensor data, upon detecting that the first module and the second module are attached to one another. Alternatively, or in addition, the sensing device may be configured to automatically stop operation of the gas sensor, upon detecting that the first module and the second module are not attached to one another. By providing an automatic activation and/or deactivation of the gas sensor (and/or of the sensing device), the comfort of use and/or the energy efficiency of the sensing device may be improved.

The sensing device may be designed such that the sensing device itself forms a lid for covering the receptacle of the storage container. The lid may comprise a flexible outer section (e.g. a rubber section) which is configured to adapt the shape and/or the diameter of the lid to the shape and/or the diameter of a plurality of different types of receptacles having different shapes and/or diameters. By providing a lid with a flexible outer section, the sensing device may be adapted in a flexible manner to different types of receptacles, thereby increasing the flexibility of use of the sensing device and/or reducing the cost of use of the sensing device.

The lid may comprise an inner section which is surrounded by the outer section (and which may be stiff or inflexible). The inner section may exhibit or comprise a pocket which is configured to receive the gas sensor in an interchangeable manner. By providing a sensing device with means for removing and/or exchanging the gas sensor, the flexibility of use of the sensing device may be increased and/or the cost of use of the sensing device may be reduced.

Hence, the sensing device may be configured to be attached to a lid of the storage container for covering the receptacle and/or to the wall of the receptacle. In particular, the sensing device may comprise a fixation component, notably a magnet or a pocket, which is configured to attach the sensing device to the storage container.

The sensing device may comprise a light unit (e.g. with one or more light emitting diodes, LEDs) configured to emit a light signal. The control unit may be configured to control the light unit to emit a light signal, dependent on whether the gas sensor is activated or not, and/or dependent on the determined freshness status of the food item. Hence, a light unit may be used for providing a direct feedback on the status of the sensing device and/or on the freshness or spoilage status of the food item, which is stored within the receptacle. By doing this, the comfort of use of the sensing device may be increased.

As already indicated above, the sensing device may comprise a battery unit, which is configured to provide electrical power for operating the gas sensor, the user interface and/or the control unit. By providing a battery unit, a self-sustained operation of the sensing device may be achieved.

According to a further aspect, a further sensing device for determining the freshness or spoilage status of a food item at least partially placeable or at least partially placed within the receptacle of a storage container is described. It should be noted that all the features described within the present document are also applicable to this sensing device (alone or in combination with one another).

The sensing device comprises a first module comprising a gas sensor, which is configured to capture sensor data regarding gas comprised within the receptacle. Furthermore, the sensing device comprises a second module comprising a control unit, which is configured to enable determination of the freshness status of the food item based on the sensor data provided by the gas sensor.

The first module and the second module are (physically) coupled with one another via a flexible strap, which is configured to be placed over the rim of the receptacle such that the first module is placed inside the receptacle and the second module is placed outside the receptacle. Hence, a sensing device is described which is attachable to a storage container in a flexible and comfortable manner, thereby allowing the freshness status of a food item to be determined in a comfortable and efficient manner.

It should be noted that the devices including their preferred embodiments as outlined in the present document may be used stand-alone or in combination with the other devices disclosed in this document. In addition, the features outlined in the context of a system are also applicable to a corresponding method. Furthermore, all aspects of the devices outlined in the present document may be arbitrarily combined. In particular, the features of the claims may be combined with one another in an arbitrary manner.

The invention is explained below in an exemplary manner with reference to the accompanying drawings, wherein
Figure 1 shows an example storage container with a volatile organic compounds (VOC) sensor, which is not part of the claimed invention;
Figures 2a to 2c show an example sensing device forming a flexible lid for a storage container, which is not part of the claimed invention;
Figures 3a to 3c show an example sensing device comprising a user interface for selecting a food item type; and
Figures 4a to 4b show an example sensing device with a strap for fixing the sensing device to a storage container.

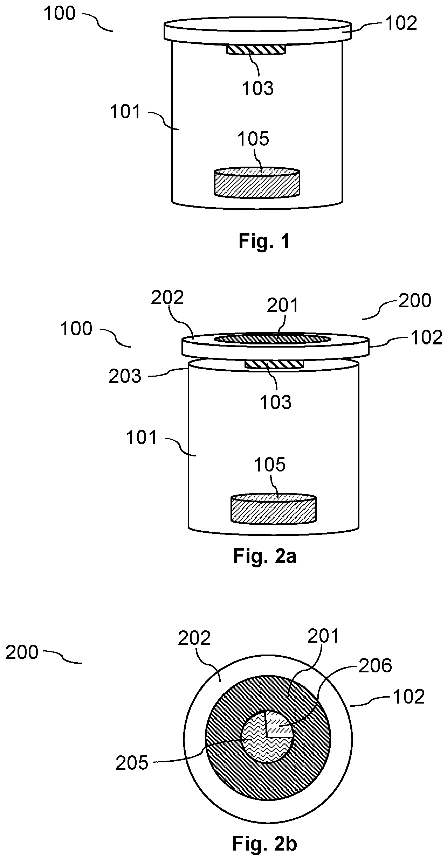

As outlined above, the present document is directed at providing means for determining the spoilage or freshness status of food that is stored within a storage container in a comfortable, efficient and reliable manner. In this context Fig. 1 shows an example storage container 100, which is not part of the claimed invention and which comprises a receptacle 101 within which food or a food item 105 may be placed. The receptacle 101 may be coved by a lid 102. Furthermore, the storage container 100 may comprise a volatile organic compounds (VOC) sensor 103 which is configured to capture sensor data regarding the gas comprised within the storage container 100. The sensor data may be analyzed to determine the spoilage or freshness status of the food item 105 that is stored within the storage container 100.

The VOC sensor 103 may be attached to the lid 102 of the storage container 100, such that the VOC sensor 103 is only usable for a single type of receptacle 101 (having a shape that corresponds to the shape of the lid 102). As a result of this, the use of a VOC sensor 103 is relatively costly, inflexible and uncomfortable.

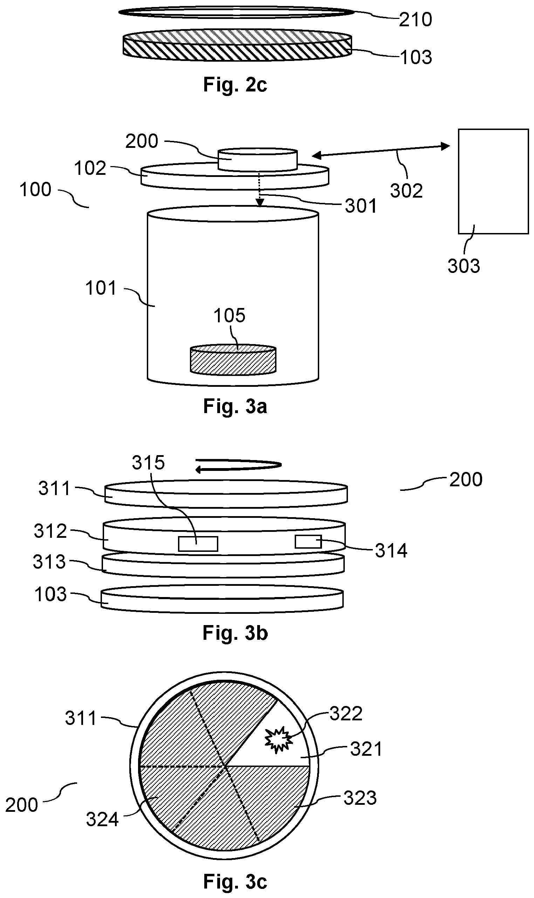

Figures 2a to 2c show different views of a sensing device 200 which comprises a VOC sensor 103. The sensing device 200 is implemented as a lid 102 with a flexible outer section 202 that allows the lid 102 to be attached to the rim 203 of differently formed and/or differently sized receptacles 101. The flexible outer section 202 may comprise an elastic material or fabric, such as rubber, which allows the outer section 202 to be stretched into different sizes and/or shapes. As a result of this, the sensing device 200 may be used as a lid 102 for a variety of different receptacles 101.

The sensing device 200 further comprises an inner section 201, which is preferably inflexible (and which is surrounded by the outer section 202). The inner section 201 comprises a pocket 205 with an opening 206 through which a VOC sensor 103 may be placed within the pocket 205. The pocket 205 is positioned at the side of the inner section 201 which is facing the interior of the storage container 100, when the lid 102 is placed on the receptacle 101. The pocket 205 may comprise a magnet 210, e.g. a magnetic ring, that is fixed to the pocket 205. The magnet 210 may be configured to hold the VOC sensor 103 at a fixed position within the pocket 205. The pocket 205 may be designed such that the VOC sensor 103 may be exchanged by a user of the sensing device 200.

Hence, the sensing device 200 may be designed such that it enables a user to remove and/or to replace the VOC sensor 103 in a comfortable manner. By doing this, a VOC sensor 103 may be used for different storage containers 100 in a flexible manner, thereby reducing the overall cost of monitoring the spoilage or freshness status of food items 105.

In other word, a sensing device 200 forming a flexible lid 102 with a VOC sensor 103 is described. The flexible and/or stretchable lid 102 is configured such that is it placable on different types of receptacles 101, such as cans, wide bottles, pots, etc. A user may place a food item 105 in a selected type of receptacle 101 and may place the flexible lid 102, notably the flexible outer section 202 of the lid 102, around the rim 203 of the receptacle 101 and push the lid 102 with the VOC sensor 103 onto the receptacle 101.

The VOC sensor 103 may be configured to sense the emitted gases periodically (e.g. every 30 minutes). Furthermore, the VOC sensor 103 may be configured to send the sensor data over a wireless communication interface (e.g. Wifi), e.g. to a database or server. The sensor data may be analyzed and the user may be alerted (e.g. via the smartphone of the user), if it is determined that the food item 105 is going to be spoiled or is over ripe. The user may then take an appropriate action.

Hence, the sensing device 200 which is described herein may comprise a flexible and/or stretchable lid 102. The outer cover of the flexible lid 102 may comprise two parts (i.e. it may be hybrid). The center section 201 of the lid 102 may be made of a hard-shell fabric. The fabric may be stiff but breathable so as to allow for a ventilation flow into and/or out of the storage container 100. The use of a hard-shell fabric allows the lid 102 to be durable.

The outer section 202 of the lid 102 may be made of a soft-shell, which is more flexible and stretchable then the center section 201. Both parts of the lid 102 may be glued (typically not woven) together, such that the gases within the interior of the storage container 100 cannot escape freely (but only in a controlled manner through the breathable fabric).

The inner side of the flexible lid 102 comprises a pocket 205 for placing the VOC sensor 103. The inside pocket 205 may comprise a magnetic ring 210 to which the VOC sensor 103 latches when placed within the pocket 205. The use of a magnet 210 allows the VOC sensor 103 to be fixed at a defined position at the lid 102. The sensing device 200 comprises the VOC sensor 103 and may comprise one or more electronic components for enabling the transmission of the sensor data via a wireless communication interface, such as WLAN.

The VOC sensor 103 may be positioned at the lid 102 such that the VOC sensor 103 may be turned on or off by a user by pressing the top of the VOC sensor 103 (which may e.g. be accessible from the outer side of the lid 102). Once the VOC sensor 103 is activated, it senses sensor data regarding the gases comprised within the interior of the storage container 100. The sensor data may be transmitted to an external entity (e.g. a server or the smartphone of a user). The sensor data may be analyzed, and based on the analysis, an alert may be provided to the user to consume the food item 105 and/or a recommendation may be provided on how to cook the particular food item 105.

As indicated above, the flexible lid 102 may be configured to be used for a variety of differently shaped or sized receptacles 101. The flexible lid 102 may be reusable. For this purpose, the flexible lid 102 may be washable and it may be composed of one or more multiple use materials. By providing a pocket 205 for a VOC sensor 103, the VOC sensor 103 may be replaced and/or upgraded in a flexible and comfortable manner.

The gases within the interior of a storage container 100 typically depend on the type of food item 105 which is placed within the storage container 100. As a result of this, the sensor data which is provided by a VOC sensor 103 typically depends on the type of food item 105. In order to allow for a precise analysis of the status of the food item 105, the type of food item 105 should be taken into account, when analyzing the sensor data of the VOC sensor 103.

Figures 3a to 3c show a sensing device 200 which comprises a user interface 311 that allows a user to specify the type of the food item 105 which is placed within the storage container 100. The user interface 311 may comprise a twist dial which is configured to be twisted or rotated by the user into different positions to select different pre-defined types of food items 105. As shown in Fig. 3c, the twist dial comprises a turning wheel 323 with a transparent segment 321 which may be placed onto different type segments 324 for different pre-defined types of food items 105. The different type segments 324 may each show a symbol 322 indicating the type of food item 105 that is associated with the type segment 324.

A user may rotate the transparent section 321 of the wheel 323 to a particular type segment 324 for selecting the corresponding type of food item 105. A control unit 315 of the sensing device 200 is configured to determine the position of the wheel 323, in order to determine the type of food item 105 that has been selected by the user. Furthermore, the control unit 315 may be configured to send information regarding the selected type of food item 105 to an external computing entity 303 over a wireless communication interface 302. For this purpose, the sensing device 200 may comprise a communication unit 314.

The sensing device 200 may be configured to be attached to the outer surface of the lid 102 of a storage container 100 (e.g. using a magnet). As shown in Fig. 3b, the sensing device 200 may comprise the user interface 311, notably a twist dial, an electronic component 312 comprising the control unit 315, the communication unit 314 and/or a battery for storing electrical power for operation of the sensing device 200. In addition, the sensing device 200 may comprise a fixation component 313 (comprising e.g. a magnet), which allows the sensing device 200 to be attached to the lid 102 of a storage container 100. In addition, the sensing device 200 comprises a VOC sensor 103. All the components shown in Fig. 3b may be integrated within a single housing.

Hence, a sensing device 200 is described, which may be implemented as a detachable dial with an embedded VOC sensor 103. Furthermore, the sensing device 200 may comprise a light unit (e.g. an LED, light emitting diode) configured to indicate the status of a food item 105. The sensor 103 and/or the light unit may be powered by a battery unit. The user may place a food item 105 into a spoilage box 100 and may rotate dial, notably the wheel 323, to select the type of the food item 105. The user interface 311 of the sensing device 200 may be configured such that the wheel 323 may be pressed by a user to indicate that the selection of the type of the food item 105 has been terminated. An identifier (ID) regarding the selected type of food item 105 may then be sent to an external entity 303 (such as a server or smartphone).

The sensing device 200 allows a user to select an appropriate type of food item 105 directly at the sensing device 200. As a result of this, a selection of the food item type at a different device (such as the smartphone of the user) is not necessary. By doing this, a particularly comfortable and efficient use of a VOC sensor 103 is enabled.

The sensing device 200 comprises a modular and replaceable twist dial and is configured to be attached to a spoilage box 100. The twist dial comprises a scroll wheel 323 which may be rotated to different segments 324 with different symbols 322 for different types of food items 105 (e.g. fruits, vegetables, fish and/or meat). The sensing device 200 comprises an electronics module 312 with a wifi communication unit 314 and a battery unit. Furthermore, the sensing device 200 comprises a VOC sensor 103 (at the bottom of the device 200).

The user may place a food item 105 in the spoilage box 100. Furthermore, the user may place the sensing device 200 on the lid 102 of the spoilage box 100, scroll through various types of food items 105 and press a center button 325 to select a particular type of food item 105 and to send an identifier regarding the selected type of food item 105 to an external entity 303 (e.g. a cloud server).

As soon as the user presses the center button 325 on the sensing device 200, the user enables the (periodic) spoilage verification of the food item 105 that is placed within the container 100 (by activation of the VOC sensor 103). Information regarding the spoilage status of the food item 105 may be provided to a smart device of the user. The sensing device 200 may send the sensor data of the VOC sensor 103 over a wireless communication interface 302 to the external entity 303. The external entity 303 may be configured to analyze the sensor data in dependence of the selected type of the food item 105. If it is determined that the food item 105 placed inside the box 100 is going to be spoiled soon, an alert may be sent to the smart device of the user. Alternatively, or in addition, a recommendation on how to cook the food item 105 may be provided.

The sensing device 200 allows a user to specify the type of the food item 105 which is placed within a storage container 100 in a comfortable and efficient manner (notably without the need of using a smart phone camera or a smartphone application or a voice command, etc., to indicate the type of the food item 105). The sensing device 200 is detachable and may be used in conjunction with various different storage containers 100. Various different numbers of different types of food items 105 may be selectable. In particular, a user may be enabled to adapt the number of type segments 324 and/or the association between the different type segments 324 and possible types of food items 105 (e.g. using an application on a smart device of the user).

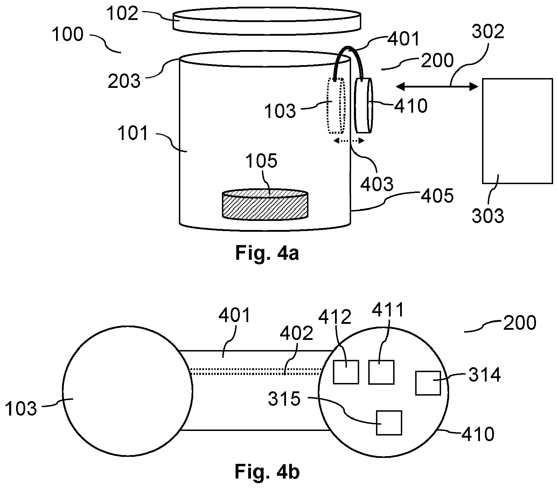

As illustrated in Figures 4a and 4b, the sensing device 200 comprises a control module 410 which is attached to the VOC sensor 103 via a strap 401. The strap 401 may comprise one or more wires 402 which are configured to connect the VOC sensor 103 to the control module 410 for providing electrical power to the VOC sensor 103 and/or for providing the sensor data from the VOC sensor 103 to the control module 410. The control module 410 may comprise the control unit 315, a communication unit 314, a battery unit 412 (for storing electrical power) and/or a light unit 411 configured to emit a light signal. The control unit 410 is referred to in the present document also as the "second module" of the sensing device 200. The VOC sensor 103 may form or may be part of a "first module" of the sensing device 200.

The strap 401 is designed such that the strap 401 allows the sensing device 200 to be placed over the rim 203 of a receptacle 101, with the VOC sensor 103 on one end of the strap 401 being at the inside of the receptacle 101 and with the control module 410 at the other end of the strap 401 being at the outside of the receptacle 101. The VOC sensor 103 and the control module 410 may be configured to exert a magnetic force 403 onto one another, such that the VOC sensor 103 at the inside of the receptacle 101 is attracted or fixed to the control module 410 at the outside of the receptacle 101. As a result of this, the sensing device 200 may be fixed to a wall 405 of the receptacle 101 in a reliable and efficient manner.

Hence, a sensing device 200 is described which comprises a VOC sensor module 103 and an associated control module 410, which are configured to be connected magnetically with one another. In particular, the sensing device 200 comprises a magnetically detachable sensor 103 and a control module 410 which are connected through a thin and durable polymer film 401. The sensor 103 and components of the control module 410 may be powered by a battery unit 412 comprised within the control module 410.

The user may place the control module 410 and the sensor module 103 next to each other and both modules 103, 410 may be attached with one another magnetically. As soon as both modules 103, 410 are attached, the sensor 103 may start to function and may send out sensor data periodically.

The wires 402 across the strap 401 between the control module 410 and the sensor module 103 may be implemented using (invisible) conductive ink. The control module 410 may be placed outside of the box 101 and the sensor module 103 may be placed inside of the box 101. The strap 401 may then run across the rim 203 of the box 101. The control module 410 and the sensor module 103 may be attached magnetically with one another (across the wall 405 of the box 101) to form a secure connection. As soon as both modules 103, 410 are attached, the sensor 103 may automatically be switched on, to sense and provide sensor data regarding the gas within the box 101.

The sensing device 200 may be used in a flexible manner with different types of storage containers 100. The strap 401 ensures that the different modules 103, 410 of the sensing device 200 are attached to one another. The strap 401 is preferably sufficiently thin to allow the lid 102 of a storage container 100 to be closed.

It should be noted that the description and drawings merely illustrate the principles of the claimed sensor device. Those skilled in the art will be able to implement various arrangements that, although not explicitly described or shown herein, embody the principles of the invention if they are within the scope of the invention defined by the appended claims. Furthermore, all examples and embodiment outlined in the present document are principally intended expressly to be only for explanatory purposes to help the reader in understanding the principles of the proposed methods and systems.

## Claims

1. A sensing device (200) for determining a freshness status of a food item (105), the food item being placed within a receptacle (101) of a storage container (100), wherein the sensing device (200) comprises
- a gas sensor (103) configured to capture sensor data regarding gas comprised within the receptacle (101);
- a user interface (311) configured to allow a user to indicate a type of the food item (105) placed within the receptacle (101); and
- a control unit (315) configured to enable determination of the freshness status of the food item (105) based on the sensor data provided by the gas sensor (103) and based on the indication of the type of the food item (105) provided by the user interface (311);
- wherein the user interface (311) is provided directly at the sensing device and the sensing device is designed as an independent device, which is configured to be operated in a stand-alone manner;
**characterized in that**
- the sensing device (200) comprises a first module comprising the gas sensor (103) and a second module (410) comprising the control unit (315) and the user interface (311);
- the user interface (311) comprises a rotatable wheel (311) configured to be turned by the user to a plurality of different type segments (324) for selecting one of a corresponding plurality of different food item types;
- the control unit (315) is configured to determine the indication of the type of the food item (105) based on the type segment (324) that the rotatable wheel (311) has been turned to; and
- the first module and the second module (410) are coupled with one another via a flexible strap (401), which is configured to be placed over a rim (203) of the receptacle (101) such that the first module is placed inside the receptacle (101) and the second module (401) is placed outside the receptacle (101).

2. The sensing device (200) of claim 1, wherein
- the sensing device (200) comprises a communication unit (314) configured to provide a communication interface (302), notably a wireless communication interface, to a remote computing entity (303); and
the control unit (315) is configured to transmit the sensor data and the indication of the type of the food item (105) via the communication interface (302), in order to enable the remote computing entity (303) to determine the freshness status of the food item (105).

3. The sensing device (200) of any previous claim, wherein the plurality of different type segments (324) comprise a plurality of different symbols (322) for indicating the corresponding plurality of different food item types.

4. The sensing device (200) of any previous claim, wherein
- the sensing device (200) comprises a button (325) configured to be actuated, notably touched and/or pushed, by the user; and
- the control unit (315) is configured to
- detect an actuation of the button (325); and
in reaction to this, determine the indication of the type of the food item (105) based on the type segment (324) that the rotatable wheel (311) has been turned to.

5. The sensing device (200) of any of the previous claims, wherein the sensing device (200) comprises a plurality of wires (402) within the strap (401) for transmitting the sensor data from the gas sensor (103) to the control unit (315) and/or for providing electrical power from the second module (410) to the gas sensor (103).

6. The sensing device (200) of any of the previous claims, wherein the first module and the second module (410) are configured to attract one another using a magnetic force (403) through a wall (405) of the receptacle (101), to attach the first module and the second module (410) to one another.

7. The sensing device (200) of claim 6, wherein the sensing device (200) is configured to
- detect whether or not the first module and the second module (410) are attached to one another; and
- automatically start operation of the gas sensor (103) for capturing sensor data, upon detecting that the first module and the second module (410) are attached to one another; and/or
- automatically stop operation of the gas sensor (103), upon detecting that the first module and the second module (410) are not attached to one another.

8. The sensing device (200) of any of the previous claims, wherein
- the sensing device (200) is configured to be attached to a lid (102) of the storage container (100) for covering the receptacle (101) and/or to a wall (405) of the receptacle (101); and/or
- the sensing device (200) comprises a fixation component (205, 210, 313), notably a magnet (210), configured to attach the sensing device to the storage container (100).

9. The sensing device (200) of any of the previous claims, wherein
- the sensing device (200) comprises a light unit (411) configured to emit a light signal; and
- the control unit (315) is configured to control the light unit (411) to emit a light signal, dependent on whether the gas sensor (103) is activated or not, and/or dependent on the freshness status of the food item (105).

10. The sensing device (200) of any of the previous claims, wherein the sensing device (200) comprises a battery unit (412) configured to provide electrical power for operating the gas sensor (103), the user interface (311) and the control unit (314), for a self-sustained operation of the sensing device (200).

## Patentansprüche

1. Erkennungsvorrichtung (200) zum Bestimmen eines Frischezustands eines Lebensmittels (105), das in einem Gefäß (101) eines Lagerbehälters (100) angeordnet ist, wobei die Erkennungsvorrichtung (200) Folgendes umfasst:
- einen Gassensor (103), der so konfiguriert ist, dass er Sensordaten zu in dem Gefäß (101) befindlichem Gas erfasst,
- eine Benutzerschnittstelle (311), die so konfiguriert ist, dass sie es einem Benutzer ermöglicht, eine Art des in dem Gefäß (101) angeordneten Lebensmittels (105) anzugeben, und
- eine Steuereinheit (315), die so konfiguriert ist, dass sie auf der Grundlage der vom Gassensor (103) gelieferten Sensordaten und der von der Benutzerschnittstelle (311) gelieferten Angabe der Art Lebensmittel (105) eine Bestimmung des Frischezustands des Lebensmittels (105) ermöglicht,
- wobei die Benutzerschnittstelle (311) direkt an der Erkennungsvorrichtung positioniert und die Erkennungsvorrichtung als unabhängige Vorrichtung ausgelegt ist, die so konfiguriert ist, dass sie autark betrieben werden kann,
**dadurch gekennzeichnet, dass**
- die Erkennungsvorrichtung (200) ein erstes Modul mit dem Gassensor (103) und ein zweites Modul (410) mit der Steuereinheit (315) und der Benutzerschnittstelle (311) umfasst,
- die Benutzerschnittstelle (311) ein drehbares Rad (311) umfasst, das so konfiguriert ist, dass es vom Benutzer zum Auswählen einer von mehreren verschiedenen entsprechenden Lebensmittelarten zu mehreren verschiedenen Artsegmenten (324) gedreht werden kann,
- die Steuereinheit (315) so konfiguriert ist, dass sie auf der Grundlage des Artsegments (324), zu dem das drehbare Rad (311) gedreht wurde, die Angabe zur Art des Lebensmittels (105) bestimmt, und
- das erste und das zweite Modul (410) über ein flexibles Band (401) miteinander verbunden sind, das so konfiguriert ist, dass es sich so auf einem Rand (203) des Gefäßes (101) anordnen lässt, dass das erste Modul in dem Gefäß (101) und das zweite Modul (401) außerhalb des Gefäßes (101) angeordnet ist.

2. Erkennungsvorrichtung (200) nach Anspruch 1, wobei:
- die Erkennungsvorrichtung (200) eine Kommunikationseinheit (314) umfasst, die so konfiguriert ist, dass sie eine Kommunikationsschnittstelle (302), insbesondere eine Drahtloskommunikationsschnittstelle, für eine entfernt gelegene Rechenfunktionseinheit (303) bereitstellt, und
die Steuereinheit (315) so konfiguriert ist, dass sie die Sensordaten und die Angabe zur Art des Lebensmittels (105) über die Kommunikationsschnittstelle (302) überträgt und so die entfernt gelegene Rechenfunktionseinheit (303) in die Lage versetzt, den Frischezustand des Lebensmittels (105) zu bestimmen.

3. Erkennungsvorrichtung (200) nach einem der vorhergehenden Ansprüche, wobei die mehreren verschiedenen Artsegmente (324) mehrere verschiedene Symbole (322) umfassen, die die jeweiligen mehreren verschiedenen entsprechenden Lebensmittelarten angeben.

4. Erkennungsvorrichtung (200) nach einem der vorhergehenden Ansprüche, wobei:
- die Erkennungsvorrichtung (200) eine Taste (325) umfasst, die so konfiguriert ist, dass sie sich vom Benutzer betätigen, insbesondere berühren und/oder drücken lässt, und
- die Steuereinheit (315) so konfiguriert ist, dass sie:
- eine Betätigung der Taste (325) erkennt und
als Reaktion darauf auf der Grundlage des Artsegments (324), zu dem das drehbare Rad (311) gedreht wurde, die Angabe zur Art des Lebensmittels (324) bestimmt.

5. Erkennungsvorrichtung (200) nach einem der vorhergehenden Ansprüche, wobei die Erkennungsvorrichtung (200) in dem Band (401) mehrere Drähte (402) zum Übertragen der Sensordaten vom Gassensor (103) zur Steuereinheit (315) und/oder zum Bereitstellen von elektrischem Strom vom zweiten Modul (410) für den Gassensor (103) umfasst.

6. Erkennungsvorrichtung (200) nach einem der vorhergehenden Ansprüche, wobei das erste und das zweite Modul (410) so konfiguriert sind, dass sie sich mithilfe einer Magnetkraft (403) durch eine Wand (405) des Gefäßes (101) hindurch gegenseitig anziehen und so aneinander befestigt sind.

7. Erkennungsvorrichtung (200) nach Anspruch 6, wobei die Erkennungsvorrichtung (200) so konfiguriert ist, dass sie
- erkennt, ob das erste und das zweite Modul (410) aneinander befestigt sind, und
- wenn sie erkennt, dass das erste und das zweite Modul (410) aneinander befestigt sind, den Gassensor (103) zum Erfassen von Sensordaten automatisch in Betrieb nimmt, und/oder
- wenn sie erkennt, dass das erste und das zweite Modul (410) nicht aneinander befestigt sind, den Betrieb des Gassensor (103) automatisch einstellt.

8. Erkennungsvorrichtung (200) nach einem der vorhergehenden Ansprüche, wobei:
- die Erkennungsvorrichtung (200) so konfiguriert ist, dass sie sich zum Abdecken des Gefäßes (101) an einem Deckel (102) des Lagerbehälters (100) und/oder einer Wand (405) des Gefäßes (101) befestigen lässt, und/oder
- die Erkennungsvorrichtung (200) eine Fixierkomponente (205, 210, 313), insbesondere einen Magneten (210), umfasst, die so konfiguriert ist, dass sie die Erkennungsvorrichtung an dem Lagerbehälter (100) befestigt.

9. Erkennungsvorrichtung (200) nach einem der vorhergehenden Ansprüche, wobei:
- die Erkennungsvorrichtung (200) eine Lichteinheit (411) umfasst, die so konfiguriert ist, dass sie ein Lichtsignal aussendet, und
- die Steuereinheit (315) so konfiguriert ist, dass sie bewirkt, dass die Lichteinheit (411) je nachdem, ob der Gassensor (103) aktiviert ist oder nicht, und/oder je nach dem Frischezustand des Lebensmittels (105) ein Lichtsignal aussendet.

10. Erkennungsvorrichtung (200) nach einem der vorhergehenden Ansprüche, wobei die Erkennungsvorrichtung (200) eine Batterieeinheit (412) umfasst, die so konfiguriert ist, dass sie für einen eigenständigen Betrieb der Erkennungsvorrichtung (200) elektrischen Strom zum Betreiben des Gassensors (103), der Benutzerschnittstelle (311) und der Steuereinheit (314) bereitstellt.

## Revendications

1. Dispositif de détection (200) pour déterminer un état de fraîcheur d'une denrée alimentaire (105), la denrée alimentaire étant placée à l'intérieur d'un réceptacle (101) d'un récipient de stockage (100),
dans lequel le dispositif de détection (200) comprend :
- un capteur de gaz (103) configuré pour capturer des données de capteur concernant des gaz compris à l'intérieur du réceptacle (101),
- une interface utilisateur (311) configurée pour permettre à un utilisateur d'indiquer un type de denrée alimentaire (105) placée dans le réceptacle (101), et
- une unité de commande (315) configurée pour permettre une détermination de l'état de fraîcheur de la denrée alimentaire (105) sur la base des données de capteur fournies par le capteur de gaz (103) et sur la base de l'indication du type de denrée alimentaire (105) fournie par l'interface utilisateur (311),
- dans lequel l'interface utilisateur (311) est agencée directement au niveau du dispositif de détection et le dispositif de détection est conçu sous forme d'un dispositif indépendant, qui est configuré pour fonctionner de manière autonome,
**caractérisé en ce que**
- le dispositif de détection (200) comprend un premier module comprenant le capteur de gaz (103) et un deuxième module (410) comprenant l'unité de commande (315) et l'interface utilisateur (311),
- l'interface utilisateur (311) comprend une molette rotative (311) configurée pour être tournée par l'utilisateur vers une pluralité de segments de type différent (324) pour sélectionner l'un d'une pluralité correspondante de types différents de denrée alimentaire,
- l'unité de commande (315) est configurée pour déterminer l'indication du type de la denrée alimentaire (105) en fonction du segment de type (324) sur lequel la molette rotative (311) a été tournée,
- le premier module et le deuxième module (410) sont couplés l'un avec l'autre via une bride flexible (401), qui est configurée pour être placée sur un rebord (203) du réceptacle (101) de sorte que le premier module est placé à l'intérieur du réceptacle (101) et le deuxième module (401) est placé à l'extérieur du réceptacle (101).

2. Dispositif de détection (200) selon la revendication 1, dans lequel :
- le dispositif de détection (200) comprend une unité de communication (314) configurée pour fournir une interface de communication (302), notamment une interface de communication sans fil, vers une entité de calcul à distance (303), et l'unité de commande (315) est configurée pour transmettre les données de capteur et l'indication du type de la denrée alimentaire (105) via l'interface de communication (302), afin de permettre à l'entité de calcul à distance (303) de déterminer l'état de fraîcheur de la denrée alimentaire (105).

3. Dispositif de détection (200) selon l'une quelconque des revendications précédentes, dans lequel la pluralité des segments de type différent (324) comprennent une pluralité de symboles différents (322) pour indiquer la pluralité correspondante des différents types de denrée alimentaire.

4. Dispositif de détection (200) selon l'une quelconque des revendications précédentes, dans lequel :
- le dispositif de détection (200) comprend un bouton (325) configuré pour être actionné, notamment touché et/ou poussé par l'utilisateur, et
- l'unité de commande (315) est configurée pour :
- détecter un actionnement du bouton (325), et en réaction à celui-ci, déterminer l'indication du type de denrée alimentaire (105) en fonction du segment de type (324) au niveau duquel la molette rotative (311) a été tournée.

5. Dispositif de détection (200) selon l'une quelconque des revendications précédentes, dans lequel le dispositif de détection (200) comprend une pluralité de fils (402) à l'intérieur de la bride (401) pour transmettre les données de capteur du capteur de gaz (103) à l'unité de commande (315) et/ou pour fournir de l'énergie électrique du deuxième module (410) au capteur de gaz (103).

6. Dispositif de détection (200) selon l'une quelconque des revendications précédentes, dans lequel le premier module et le deuxième module (410) sont configurés pour s'attirer l'un l'autre en se servant d'une force magnétique (403) à travers une paroi (405) du réceptacle (101), afin d'associer le premier module et le deuxième module (410) l'un à l'autre.

7. Dispositif de détection (200) selon la revendication 6, dans lequel le dispositif de détection (200) est configuré pour :
- détecter si oui ou non le premier module et le deuxième module (410) sont associés l'un à l'autre, et
- démarrer automatiquement le fonctionnement du capteur de gaz (103) pour capturer des données de capteur lorsqu'il est détecté que le premier module et le deuxième module (410) sont associés l'un à l'autre, et/ou
- stopper automatiquement le fonctionnement du capteur de gaz (103) lorsqu'il est détecté que le premier module et le deuxième module (410) ne sont pas associés l'un à l'autre.

8. Dispositif de détection (200) selon l'une quelconque des revendications précédentes, dans lequel :
- le dispositif de détection (200) est configuré pour être fixé à un couvercle (102) du récipient de stockage (100) pour recouvrir le réceptacle (101) et/ou à une paroi (405) du réceptacle (101), et/ou
- le dispositif de détection (200) comprend un élément de fixation (205, 210, 313), notamment un aimant (210), configuré pour fixer le dispositif de détection au récipient de stockage (100).

9. Dispositif de détection (200) selon l'une quelconque des revendications précédentes, dans lequel :
- le dispositif de détection (200) comprend un dispositif lumineux (411) configuré pour émettre un signal lumineux, et
- l'unité de commande (315) est configurée pour commander le dispositif lumineux (411) afin d'émettre un signal lumineux en fonction du fait que le capteur de gaz (103) est activé ou non, et/ou en fonction de l'état de fraîcheur de la denrée alimentaire (105).

10. Dispositif de détection (200) selon l'une quelconque des revendications précédentes, dans lequel le dispositif de détection (200) comprend un dispositif de batterie (412) configuré pour fournir de l'énergie électrique pour faire fonctionner le capteur de gaz (103), l'interface utilisateur (311) et l'unité de commande (314), pour assurer un fonctionnement auto-entretenu du dispositif de détection (200).
